# EUROPEAN PATENT APPLICATION

(11) **EP 1 707 197 A1**
(43) Date of publication of application: **04.10.2006**
(21) Application number: 06250763.7
(22) Date of filing: 13.02.2006
(51) Int. Cl.: A61K 31/216

(54) **Formulations containing fenofibrate and a surfactant mixture**

(30) Priority: 30.03.2005 US 666192 P; 28.12.2005 US 321157; 29.12.2005 EP 05258102
(71) Applicant: TEVA PHARMACEUTICAL INDUSTRIES LTD., 49131 Petah Tiqva (IL)
(72) Inventor: Lerner, E. Itzhak, Peach Tiqva 49404 (IL); Rosenberger, Vered, Modiin 71700 (IL); Falshner-Barak, Moshe, Petach Tiqva 49313 (IL); Drabkin, Anna, Tzur Hadassah (IL); Moldavski, Naomi, HaNegev 85492 (IL)
(74) Representative: Nachshen, Neil Jacob

(57) **Abstract**

The invention provides at least a composition for the treatment of elevated levels of triglycerides, comprising a therapeutically effective amount of a fibrate drug, preferably fenofibrate, intimately associated with a surfactant mixture, preferably a mixture comprising PGE 6000 and poloxamer 407. The invention also provides a method for the treatment of elevated levels of triglycerides in a subject, comprising administering to the subject the composition.

## Description

This application claims the benefit of U.S. Provisional Application No. 60/666,192, filed March 30, 2005, and is a continuation-in-part application of U.S. Non-Provisional Application No. (application number to be assigned) filed on December 28, 2005 under Docket No. 01662/70201 by Lerner, Rosenberger, Flashner-Barak, Drabkin and Moldavski with a title, "Pharmaceutical Formulations of Fenofibrate Having Improved Bioavailability" and its corresponding European application EP2005258102.2 filed 29 December 2005, wherein the disclosures of these earlier applications are incorporated by reference in their entirety.

### Background

Fenofibrate, (2-[4-(4-chlorobenzoyl) phenoxy]-2-methyl-propanoic acid, 1-methylethyl ester) is one of the fibrate class of drugs. It is available as both capsules and tablets. Fenofibrate is apparently a prodrug. Fenofibrate is sold by Abbott Laboratories Ltd under the trademark Tricor ® as tablets containing 48mg and 145mg¹.
¹ The Prescribing Information available from http://rxabbott.com/pdf/tricorpi.pdf describes the tablets as containing either 48mg or 145mg fenofibrate and the following inactive ingredients: hydromellose 2910 (3cps), docusate sodium, sucrose, sodium lauryl sulfate, 48mg tablet: polyvinyl alcohol, titanium dioxide, talc, soybean lecithin, xanthan gum, D&C Yellow No. 10 aluminium lake, FD&C Yellow No. 6/sunset yellow FCF aluminium lake, FD&C blue No. 2/indigo carmine aluminium lake. 145mg tablets: polyvinyl alcohol, titanium dioxide, talc, soybean lecithin, xanthan gum. Tricor 54mg tablets were approved as NDA 21-203 and described a containing as inactive ingredients: colloidal silicon dioxide, crospovidone, lactose monohydrate, lecithin, microcrystalline cellulose, polyvinyl alcohol, povidone, sodium lauryl sulfate, sodium stearyl fumarate, talc, titanium dioxide, and xanthan gum., D&C Yellow No. 10, FD&C Yellow No. 6, FD&C Blue No. 2. (www.fda.gov/cder/foi/label/2001/212031bl.pdf lactose monohydrate, silicified microcrystalline cellulose, crospovidone and magnesium stearate. In addition, individual tablets contain: The active moiety is reportedly the metabolite fenofibric acid which is reported to be produced in the body by esterases. When dosing fenofibrate, apparently no intact fenofibrate is found in the plasma (Physician's Desk Reference, 58th ed., 2004, pages 522 - 525 (PDR)) .

Fenofibrate is a very poorly soluble drug. Despite its poor solubility, it is reported to be well absorbed when dosed in the "fed state" and less so in the "fasted state". It is unclear what the bioavailability of the fenofibric acid really is, since much of it is understood to be metabolized to the glucuronide in both presystemic and first pass sites. The absolute bioavailability of fenofibrate cannot supposedly be determined since the compound is insoluble in media suitable for intravenous injection. Following oral administration in healthy volunteers, approximately 60% of a single dose of radiolabelled fenofibrate appeared in urine, primarily as fenofibric acid and its glucuronide conjugate, and 25% was excreted in the feces (PDR). The absorption of fenofibrate is understood to be increased when administered with food. With fenofibrate tablets, the extent of absorption is increased by approximately 35% when dosed with food (PDR, Martindale 33rd ed., page 889).

Much effort has been expended to improve the formulation of fenofibrate. U.S. Patent Nos. 4,895,726 and 5,880,148 disclose co-micronizing the fenofibrate with surface active agents. U.S. Patent Nos. 6,074,670, 6,277,405 and others disclose micronized fenofibrate coated onto hydrosoluble carriers with optional surface active agents. U.S. Patent No. 6,814,977 discloses fenofibrate dissolved in a medium chain glycerol ester of fatty acid, U.S. Patent No. 6,719,999 discloses fenofibrate dissolved in glycerin, propylene glycol, or dimethylisosorbide and U.S. Patent No. 5,827,536 discloses fenofibrate dissolved in diethyleneglycol monoethyl ether.

Several patents disclose specific formulations of micronized fenofibrate with specific polymeric or surface active agent additives while several others describe emulsions and suspensions. U.S. Patent Application Publication No. 20040087656 discloses fenofibrate of particle size less than 2000 nm with an improved bioavailability. U.S. Patent Application Publication No. 20030224059 discloses microparticles of active pharmaceutical ingredients, drug delivery vehicles comprising same, and methods for making them. The disclosure of US 20030224059 is incorporated herein by reference in its entirety.

U.S. Patent Application Publication No. 20040198646 discloses compositions comprising solutions of drugs in menthol, especially drugs that are poorly soluble in water, and to methods for making such compositions. The disclosure of US 20040198646 is incorporated in its entirety by reference.

Micronization of the drug and the addition of surface active agents have moderately raised the bioavailability of fenofibrate allowing the amount of drug dosed to be reduced from 100 mg per dose to 67 mg per dose and then subsequently to 54 mg per dose, all with the same bioavailability in the fed state. Nanoparticle formulations of the drug have further allowed the reduction of the dose to 48 mg per dose with the bioavailability of the "fasted state" being reported as similar to the fed state. There is still room for much improvement since it is posited that the true bioavailability of fenofibrate is still relatively low.
The current inventors have surprisingly found that a composition of fenofibrate dissolved in menthol and comprising surface active agents gives a much enhanced bioavailability well beyond anything previously disclosed. The inventors have also surprisingly found formulations with or without menthol of increased solubility and drug release of fenofibrate

### Summary of the Invention

The present invention encompasses a composition for the treatment of elevated levels of triglycerides that comprises a therapeutically effective amount of fenofibrate or another fibrate drug that is intimately associated with menthol. The intimate association may be in the form of a solution of the fenofibrate or other fibrate in menthol but would encompass compositions where at least part of the drug has come out of such a solution due to a process that induces the precipitation of the drug, e.g. saturation such as reducing the volume of the solvent or cooling. The composition may be optionally absorbed in, or adsorbed on a solid carrier by methods exemplified by the teachings in US 2003-0224059 and US 2004-0198646.

The present invention encompasses a composition for the treatment of elevated levels of triglycerides that comprises a therapeutically effective amount of fenofibrate or another fibrate drug that is dissolved in menthol and further comprises at least one surface active agent. The composition may be optionally absorbed on a solid carrier.

The present invention encompasses a composition that comprises a therapeutically effective amount of fenofibrate or another fibrate drug that is dissolved in menthol and further comprises at least one surface active agent. The composition can have a dissolution property in that, when tested in 50 ml 0.1 N HCl at 37° C and 150 rpm, at least about 10%, 30% or 80% of the fenofibrate or other fibrate drug dissolved in 15 minutes. The composition can also have a dissolution property in that, when tested in 500 ml 0.5% sodium lauryl sulfate (SLS) in water at 37° C and 50 rpm, at least about 70% of the fenofibrate or other fibrate drug dissolved in 5 minutes.

The present invention encompasses a composition that comprises a therapeutically effective amount of fenofibrate or another fibrate drug that is dissolved in menthol and further comprises at least one surface active agent that when administered orally to beagle dogs shows a bioavailability of fenofibric acid based on Area Under the Curve (AUC) of the concentration v. time profile in plasma that is at least three times that of the Tricor® 54 mg product on a per milligram basis (when normalized to equal weight).

The present invention also encompasses the method of preparing a composition of the invention, which method comprises:
a) heating menthol to about 60 °C in order to effect melting thereof,
b) adding at least one surface active agent to the melt,
c) cooling the product of step b) to about 50 °C,
d) dissolving fenofibrate or another fibrate drug in the product of step c) with stirring,
e) cooling the product of step d) to room temperature to obtain the composition of the invention, and
f) if capsules are desired, (A) dispensing the product of step e) into capsules, or (B) alternatively adding a solid carrier such as microcrystalline cellulose, lactose or sorbitol, to the product of step e), mixing well, cooling to room temperature and filling the powder thus obtained into capsules.

The present invention encompasses a composition that comprises a therapeutically effective amount of fenofibrate or another fibrate drug that is dissolved in a surfactant mixture, such as a surfactant mixture comprising polyethylene glycol and Poloxamer, e.g., a surfactant mixture comprising Polyethylene Glycol (PEG) 1000 and Poloxamer 407, or a surfactant mixture comprising PEG 6000 and Poloxamer 407. The composition may be optionally absorbed or adsorbed on a solid carrier.

The present invention encompasses a composition that comprises a therapeutically effective amount of fenofibrate or another fibrate drug that is intimately associated with a surfactant mixture, such as a surfactant mixture comprising polyethylene glycol and Poloxamer, e.g., a surfactant mixture comprising PEG 1000 and Poloxamer 407, or a surfactant mixture comprising PEG 6000 and Poloxamer 407. This composition can have a dissolution property in that, when tested in 900 ml 0.5% sodium lauryl sulfate (SLS) in water at 37 °C and 50 rpm, at least about 40% of the fenofibrate or the other fibrate drug dissolved in 15 minutes and/or about 80% dissolved in 30 minutes. The intimate association may be in the form of a solution but would encompass compositions where at least part of the drug has come out of such a solution or has not fully dissolved due to e.g. saturation. When administered orally to beagle dogs, this composition of the invention shows a bioavailability of fenofibric acid, based on Area Under the Curve (AUC) of the concentration vs. time profile in plasma, that is at least about two times that of the Tricor® 54 mg product on a per milligram basis (when normalized to equal weight).

The present invention also encompasses a method of treating a patient for elevated triglyceride levels comprising administering to the patient a composition of fenofibrate that comprises a therapeutically effective amount of fenofibrate that is dissolved in menthol.

The present invention also encompasses a method of treating a patient for elevated triglyceride levels comprising administering to the patient a composition of fenofibrate that comprises a therapeutically effective amount of fenofibrate that is dissolved in menthol and further comprises at least one surface active agent.

The present invention also encompasses a method of treating a patient for elevated triglyceride levels comprising administering to the patient a composition of fenofibrate that comprises a therapeutically effective amount of fenofibrate that is dissolved in PEG 1000 and Poloxamer 407.

The present invention also provides a method of treating a subject for elevated triglyceride levels comprising administering to the subject a composition comprising a therapeutically effective amount of fenofibrate or another fibrate drug in intimate association with a surfactant mixture comprising PEG 6000 and Poloxamer 407.

### Detailed Description of the Invention

There is a need to improve pharmaceutical compositions of fenofibrate, a drug used in treating hypertriglyceridemia. The term "fenofibrate" includes the 1-methylethyl ester of 2-[4-(4-chlorobenzoyl)-phenoxy]-2-methyl-propanoic acid and any pharmaceutically acceptable salts thereof. One aspect of this invention is to compositions of fenofibrate that is dissolved in menthol. Fenofibrate dissolves up to about 37% in melted menthol at 60°C. Formulations may be made where all the fenofibrate is dissolved in the menthol or where only some of the fenofibrate is so dissolved and the rest present in a solid form in the fully saturated menthol medium. In a preferred embodiment of the invention, the fenofibrate is fully dissolved in the menthol. In one embodiment of the invention, the menthol melt may be filled into capsules in the liquid state or may be solidified, optionally milled, and filled into capsules. The capsules used for the liquid fill in one embodiment may be hard gelatin capsules. In a preferred embodiment, the hard gelatin capsules are banded to prevent leakage. In a more preferred embodiment, the liquid formulation may be filled into soft-gel capsules. In another embodiment, the solidified menthol solution is optionally milled and filled into hard gelatin capsules or equivalent capsules of other materials such as materials of vegetable origin (e.g., HPMC). In another preferred embodiment, the melted menthol formulations may be further adsorbed on a solid carrier. Such solid carriers can be water soluble (hydrosoluble) carriers such as sucrose, lactose, mannitol or sorbitol or water insoluble carriers such as starch, cellulose, microcrystalline cellulose, or calcium phosphate. The so formed powder can optionally be mixed with standard pharmaceutical additives to help flow or other properties and can be filled into hard gelatin capsules or their equivalents. In another preferred embodiment, these powders can be optionally mixed with standard pharmaceutical excipients and formulated for tablet formation in a tablet press.

In this instant patent application, the term "another fibrate drug" or "other fibrate drug" includes fenofibric acid, any salt of fenofibric acid, any ester of fenofibric acid except the 1-methylethyl ester which is encompassed by the term "fenofibrate" as defined above, bezafibrate, binifibrate, clinofibrate, ciprofibrate, clofibrate, clofibride, etofibrate, etofylline clofibrate, gemfibrozil, pirifibrate, ronifibrate and simfibrate.

In the patent application, where fenofibrate or another fibrate drug is "intimately associated with menthol", "in intimate association with menthol", "intimately associated with a surfactant mixture" or "in intimate association with a surfactant mixture", it is intended to include
a) a solution of fenofibrate or the other fibrate drug in menthol, menthol surfactant mixture, or surfactant mixture whether the menthol, menthol mixture or surfactant mixture is a liquid, melt or solid (a solid solution);
b) a precipitate of fenofibrate or the other fibrate drug, or a co-precipitate of fenofibrate or the other fibrate drug and any additive(s) from the menthol solution, menthol surfactant mixture solution or surfactant mixture solution, which is coated by or in contact with the saturated or supersaturated solution; and/or
c) fenofibrate or the other fibrate drug coated by or in contact with a saturated solution of fenofibrate or the other fibrate drug in menthol, the menthol surfactant mixture or surfactant mixture, wherein the remaining fenofibrate or the other fibrate drug does not dissolve because the amount of fenofibrate or the other fibrate drug present is above saturation.

The expressions, "intimately associated" and "in intimate association", exclude a simple physical mixture of two solids by blending or by granulation in a granulation liquid which does not at least partially dissolve the fenofibrate or the other fibrate drug.

In this patent application, when a quantitative value is preceded with "about", it is intended to cover ± 5% of the value. For instance, "about 50%" means from 47.5% to 52.5%.

Another aspect of this invention is a composition for the treatment of elevated levels of triglycerides that comprises a therapeutically effective amount of fenofibrate that is dissolved in menthol and further comprises at least one surface active agent. Formulations may be made where all the fenofibrate is dissolved in the menthol or where only some of the fenofibrate is so dissolved and the rest present in a solid form in the fully saturated menthol medium. In a preferred embodiment, the fenofibrate is dissolved in the menthol plus surface active agent medium. In a more preferred embodiment, the fenofibrate is fully dissolved in the menthol which also comprises the surface active agent. Surface active agents that can be used with this embodiment comprise the Tweens, most preferably Tween 80, sodium ducosate, sodium lauryl sulfate, Cremophor, polyethylene glycols (PEG), preferably PEG 1000 or PEG 6000, and poloxamers, most preferably poloxamer 407. Preferred embodiments comprise by weight fenofibrate 2% to 40%, more preferably 5% to 25%, menthol 10% to 90%, more preferably 15% to 40%, and surface active agents 10% to 80%, more preferably 30% to 70%. In another preferred embodiment the melted menthol formulations may be further adsorbed on, or absorbed in, a solid carrier. Such solid carriers can be water soluble (hydrosoluble) carriers such as sucrose, lactose or sorbitol or water insoluble carriers such as starch, cellulose, microcrystalline cellulose, or calcium phosphate. The so formed powder can optionally be mixed with standard pharmaceutical additives to help flow or other properties and can be filled into hard gelatin capsules or their equivalents. In another preferred embodiment these powders can be optionally mixed with standard pharmaceutical excipients and formulated for tablet formation in a tablet press or formed into a melt tablet.

In a preferred embodiment of a composition of the invention, a formulation comprised about 25.2% fenofibrate , about 23.4% menthol, about 11.7% sodium ducosate and about 39.7 % Tween 80. When the drug release of this formulation was tested in a small volume drug release test of 50 ml 0.1N HCl at 37°C and 150 rpm, about 11.9% of the fenofibrate in the composition were dissolved in 15 minutes.

Another preferred embodiment of the composition of the invention comprises about 20.5% fenofibrate, about 37.9% menthol, about 9.5% sodium ducosate and about 32.2% Tween 80. When the drug release of this composition was tested in a small volume drug release test of 50 ml O.1N HCl at 37°C and 150 rpm, about 31.7% of the fenofibrate in the composition were dissolved in 15 minutes.

Another preferred embodiment of the composition of the invention comprises about 12.4% fenofibrate, about 18.4% menthol, and about 69.1% Tween 80. When the drug release of this composition was tested in a small volume drug release test of 50 ml 0.1N HCl at 37°C and 150 rpm, about 12.5% of the fenofibrate in the composition were dissolved in 15 minutes.

Another preferred embodiment of the composition of the invention comprises about 12.4% fenofibrate, about 18.4% menthol, and about 69.1% Cremophor. When the drug release of this composition was tested in a small volume drug release test of 50 ml 0.1N HCl at 37°C and 150 rpm, about 17.9% of the fenofibrate in the composition were dissolved in 15 minutes.

Another preferred embodiment of the composition of the invention comprises about 10.9% fenofibrate, about 16.2% menthol, about 8.1% sodium ducosate, about 4.0% glycerine and about 60.7% Cremophor. When the drug release of this composition was tested in a small volume drug release test of 50 ml 0.1N HCl at 37°C and 150 rpm, about 15.6% of the fenofibrate in the composition dissolved in 15 minutes.

In a most preferred embodiment of the composition of the invention comprises about 7.7% fenofibrate, about 19.2% menthol, about 7.7% sodium ducosate and about 65.4% Tween 80. When the drug release of this composition was tested in a small volume drug release test of 50 ml 0.1N HCl at 37°C and 150 rpm, about 93.3% of the fenofibrate in the composition dissolved in 15 minutes. This most preferred embodiment was further tested in 500 ml 0.5% sodium lauryl sulfate (SLS) in water at 37°C and 5.0 rpm where it gave a release profile of 78.7% dissolved at 5 minutes and 92.5% dissolved at 10 minutes.

By comparison, the fenofibrate composition as taught in example 2 of U.S. application 10/400,100 (US 2003/0224059) when tested in less stringent, more conducive conditions for dissolution, in a USP Apparatus II dissolution tester in 900 ml 0.5% sodium lauryl sulfate (SLS) in water at 37°C and 100 rpm, displayed a rate of dissolution such that it took approximately 90 minutes for greater than 90% of the fenofibrate to dissolve. This most preferred embodiment was further tested for its pharmacokinetic profile in dogs vs. the micronized formulation of commercial Tricor® 54 mg (see example 3) and gave a bioavailability of the active metabolite fenofibric acid that was improved by a factor of more than four on a per milligram basis.

Another aspect of this invention encompasses the method of preparing the fenofibrate menthol compositions. In one preferred embodiment, this method comprises the heating of menthol to about 50 - 70°C, most preferably about 60°C, in order to effect melting of the menthol. The menthol melt is stirred at a convenient rate. The method further comprises adding a surface active agent or more than one agent to the melt. The melt is stirred gently until a full solution has been achieved. In a preferred embodiment, the surface active agent is Tween 80. In a more preferred embodiment the surface active agent comprises both Tween 80 and Sodium ducosate. In one embodiment, fenofibrate is added to the melt at this point. In a more preferred embodiment the melt is cooled to between 45°C and 55°C, most preferably to about 50°C, before adding the fenofibrate. The melt is stirred at about the same temperature until all the fenofibrate dissolves. In one preferred embodiment the solution thus obtained is dispensed into either hard or soft capsules. More preferably the solution (or melt) is first cooled to room temperature and then dispensed into either hard or soft capsules. The hard capsules are preferably sealed by "banding" to prevent leakage. In another preferred embodiment of this method a solid carrier such as microcrystalline cellulose, lactose or sorbitol or a combination thereof is added to the melt either before or after cooling to room temperature. The mixture is mixed well, cooled to room temperature if necessary, and filled into capsules. Optionally, other excipients may be added to the powder such as flow aids. In another preferred embodiment the powder so obtained is further formulated with additives that allow it to be pressed into a tablet in a tablet press.

Another aspect of this invention relates to compositions of fenofibrate or another fibrate drug that are menthol free but comprise a therapeutically effective amount of the fenofibrate or other fibrate drug that is dissolved in, or in intimate association with a surfactant mixture comprising polyethylene glycol (PEG) and Poloxamer. PEG useful for this embodiment are all PEG's that are liquid at room temperature or that melt up to about 70°C. The most preferred PEG is PEG 1000 or PEG 6000. The most preferred Poloxamer is Poloxamer 407. In an embodiment, the composition can comprise by weight fenofibrate from about 5% to about 50%, PEG 1000 from about 5% to about 50% and Poloxamer 407 from about 5% to about 50%. In another embodiment, the composition comprises between about 15% and about 25% by weight of the fibrate drug, preferably fenofibrate, between about 7% and about 13% by weight of PEG 6000, and between about 7% and about 13% by weight of Poloxamer 407, wherein the fibrate drug preferably is fenofibrate. In yet another preferred embodiment the compositions further contain a pharmaceutically acceptable carrier. In such an embodiment, the fenofibrate may be adsorbed on or absorbed in the solid carrier. Such solid carriers can be water soluble (hydrosoluble) carriers such as sucrose, lactose or sorbitol or water insoluble carriers such as starch, cellulose, microcrystalline cellulose, or calcium phosphate. The so formed powder can optionally be mixed with standard pharmaceutical additives to help flow or other properties and can be filled into hard gelatin capsules or their equivalents. In another preferred embodiment these powders can be optionally mixed with standard pharmaceutical excipients and formulated for tablet formation in a tablet press.

The present invention also provides a composition comprising about 19% by weight of fenofibrate, about 10.9% by weight of Poloxamer 407, about 10.9% by weight of PEG 6000 by weight, about 15.3% by weight of microcrystalline cellulose, about 18% by weight of crospovidone, about 12% by weight of sodium bicarbonate and about 12% by weight of citric acid, wherein the fenofibrate is dissolved in or intimately associated with the PEG 6000 and Poloxamer 407.

The invention further provides a composition comprising about 19% by weight of fenofibrate, about 10.9% by weight of Poloxamer 407, about 10.9% by weight of PEG 6000 by weight, about 15.3% by weight of microcrystalline cellulose, about 18% by weight of crospovidone, about 12% by weight of sodium bicarbonate and about 12% by weight of tartaric acid, wherein the fenofibrate is dissolved in or intimately associated with the PEG 6000 and Poloxamer 407.

The pharmaceutical composition of the present invention comprising a fibrate drug, preferably fenofibrate, in a therapeutically effective amount, that is intimately associated with a surfactant mixture, such as polyethylene glycol and Poloxamer, e.g., PEG 6000 and Poloxamer 407, can optionally be adsorbed on or absorbed in a solid carrier. The pharmaceutical composition can have a dissolution property in that, when tested using a USP type II dissolution tester filled with 1000 ml 0.5% sodium lauryl sulfate (w/v) in water at 37 °C and 50 rpm, at least about 50%, preferably about 50-80%, e.g., about 55-76% (such as about 68%) or about 70-80%, is released in 10 minutes; at least about 73%, preferably about 73-93%, e.g., about 77-89% (such as about 83%) or about 86-93%, is released in 15 minutes; and at least about 85%, preferably about 85-99%, e.g., about 87-97% (such as about 90%) or about 93-100%, is released in 30 minutes.

The pharmaceutical composition of the present invention comprising a therapeutically effective amount of fenofibrate or another fibrate drug intimately associated with a surfactant mixture comprising PEG 6000 and Poloxamer 407 can be used to make formulations such as tablets or capsules. For tablets comprising about 145 mg fenofibrate administered orally to humans in a fed state, when the pharmacokinetics based on the plasma concentration of fenofibric acid is determined, the average area under the plasma concentration versus time curve from time zero to about 48 hours, i.e., AUC₀₋₄₈ₕ, can range from about 91600 h.ng/m to about 217500 h·ng/g (preferably the average AUC₀₋ₜ is about 150500 h.ng/g), and the average AUC from time zero to infinity, i.e., AUC_{inf}, can range from about 97200 h.ng/g to about 308100 h·ng/g (preferably the average AUC_{inf} is about 185200 h.ng/g). The geometric mean of the ratio of the AUC_{inf} for a formulation prepared with a pharmaceutical composition comprising fenofibrate intimately associated with a surfactant mixture comprising PEG 6000 and Poloxamer 407 of the present invention when orally administered to a group of fed human subjects versus the AUC_{inf} of 145 mg Tricor tablets administered orally to the group of fed human subjects is about 0.80 to about 1.25, preferably about 1, wherein the AUC_{inf} ratio is calculated on a per human subject basis, and wherein the geometric mean is calculated using the individual AUC_{inf} ratios for the human subjects in the group. Similarly, the geometric mean of the ratio of the AUC₀₋₄₈ₕ for the formulation prepared with a pharmaceutical composition comprising fenofibrate intimately associated with a mixture of PEG 6000 and Poloxamer 407 of the present invention administered orally to a group of fed human subjects versus the AUC₀₋₄₈ₕ of 145 mg Tricor tablets administered orally to the group of fed human subjects is about 0.80 to about 1.25, preferably about 1. Also similarly, the geometric mean of the ratio of Cₘₐₓ for the formulation prepared with a pharmaceutical composition comprising fenofibrate intimately associated with a surfactant mixture comprising PEG 6000 and Poloxamer 407 of the present invention administered orally to a group of fed human subjects versus the Cₘₐₓ for 145 mg Tricor tablets administered orally to the group of fed human subjects is about 0.80 to about 1.25, preferably about 1.

When the pharmaceutical composition of the present invention comprising a therapeutically effective amount of fenofibrate or another fibrate drug intimately associated with a surfactant mixture comprising PEG 6000 and Poloxamer 407 is used to make tablets comprising about 145 mg fenofibrate, and when the tablets are administered orally to humans in a fasted state with the pharmacokinetics based on the plasma concentration of fenofibric acid determined, the average AUC from time zero to about 48 hours, i.e., AUC₀₋₄₈ₕ, can range from about 121400 h·ng/g to about 287500 h·ng/g (preferably the average AUC₀₋₄₈ₕ is about 175300 h·ng/g); the average AUC from time zero to infinity, i.e., AUC_{inf}, can range from about 134800 h·ng/g to about 345400 h·ng/g (preferably the average AUC_{inf} is about 213700 h·ng/g) and the average maximum value in the plasma concentration versus time curve, i.e., Cₘₐₓ, can range from about 6400 ng/g to about 14600 ng/g (preferably the average Cₘₐₓ is 10600 ng/g). The geometric mean of the ratio of the AUC_{inf} for a formulation prepared with a pharmaceutical composition comprising fenofibrate intimately associated with a surfactant mixture comprising PEG 6000 and Poloxamer 407 of the present invention when orally administered to a group of human subjects in a fasted state versus the AUC_{inf} of 145 mg Tricor tablets administered orally to the group of human subjects in the fasted state is about 0.80 to about 1.25, preferably about 1, wherein the AUC_{inf} ratio is calculated on a per human subject basis, and wherein the geometric mean is calculated using the individual AUC_{inf} ratios for the human subjects in the group. Similarly, the geometric mean of the ratio of the AUC₀₋₄₈ₕ for the formulation prepared with a pharmaceutical composition comprising fenofibrate intimately associated with a surfactant mixture comprising PEG 6000 and Poloxamer 407 of the present invention administered orally to a group of human subjects in a fasted state versus the AUC₀₋₄₈ₕ of 145 mg Tricor tablets administered orally to the group of human subjects in the fasted state is about 0.80 to about 1.25, preferably about 1. Also similarly, the geometric mean of the ratio of Cₘₐₓ for the formulation prepared with a pharmaceutical composition comprising fenofibrate intimately associated with a surfactant mixture comprising PEG 6000 and Poloxamer 407 of the present invention administered orally to a group of human subjects in a fasted state versus the Cₘₐₓ for 145 mg Tricor tablets administered orally to the group of human subjects in the fasted state is about 0.80 to about 1.25, preferably about 1.

The present invention also provides a process for preparing the pharmaceutical composition of the present invention comprising fenofibrate or another fibrate drug, preferably fenofibrate, in a therapeutically effective amount, that is intimately associated with a surfactant mixture, which process comprises:
(a) providing melted menthol;
(b) mixing melted menthol with the fenofibrate or other fibrate drug and a surfactant mixture comprising polyethylene glycol and Poloxamer to dissolve at least part of the fenofibrate or other fibrate drug and the surfactant mixture comprising polyethylene glycol and Poloxamer in the menthol; and
(c) removing the menthol via sublimation to obtain a mixture as the pharmaceutical composition,
wherein preferably the polyethylene glycol is PEG 6000 and the Poloxamer is Poloxamer 407;
wherein optionally the mixing step (b) includes the addition of at least one other pharmaceutically acceptable carrier or excipient, and/or a pharmaceutically acceptable solid carrier; and
wherein step (c) is preferably conducted by applying a vacuum such as 0.2 mbar to the product of step (b).

In a preferred embodiment the formulation comprises about 12.4% fenofibrate, about 18.4% PEG 1000, and about 69.1 % Poloxamer 407. When this embodiment was tested in 900 ml 0.5% sodium lauryl sulfate (SLS) in water at 37° C and 50 rpm, 79.4% of the fenofibrate were dissolved at 15 minutes, 84.6% were dissolved at 30 minutes and 85.2% were dissolved at 60 minutes. Micronized fenofibrate, tested under the same conditions gave corresponding results of 10.7% for 15 minutes 20.2% for 30 minutes and 31.6% for 60 minutes.

Another preferred embodiment comprises about 35.1% fenofibrate, about 32.5% PEG 1000, and about 32.5% Poloxamer 407. When this embodiment was tested in 900 ml 0.5% sodium lauryl sulfate (SLS) in water at 37° C and 50 rpm, 41.8% of the fenofibrate were dissolved at 15 minutes, 84.9% were dissolved at 30 minutes and 91.8% were dissolved at 60 minutes. Micronized fenofibrate, tested under the same conditions gave corresponding results of 10.7% for 15 minutes 20.2% for 30 minutes and 31.6% for 60 minutes.

A most preferred embodiment comprises about 9.9% fenofibrate, about 6.6% PEG 1000, about 1.0% sodium ducosate, about 6.6% Gelucire® 33/01 and about 9.9% Poloxamer 407, all adsorbed on the solid carrier sorbitol which comprised about 66% of the weight. This preferred embodiment could be delivered in a capsule or more preferably pressed into tablet form. When this embodiment was tested in 500 ml 0.5% sodium lauryl sulfate (SLS) in water at 37° C and 50 rpm, 18.4% of the fenofibrate were dissolved at 5 minutes, 47.2% were dissolved at 10minutes, 70.9% were dissolved at 20 minutes and 78.0% were dissolved at 30 minutes. This most preferred embodiment of this aspect of the invention was further tested for its pharmacokinetic profile in dogs vs. the micronized formulation of commercial Tricor® 54 mg (see example 3) and gave a bioavailability of the active metabolite fenofibric acid that was more than two times better on a per milligram basis.

Another aspect of this invention encompasses the method of treating a patient for elevated triglyceride levels comprising administering to the patient a composition of fenofibrate that comprises a therapeutically effective amount of fenofibrate that is dissolved in menthol. In one embodiment the drug is dosed as a viscous solution in a capsule. In one embodiment this capsule is a hard gelatin capsule or equivalent. In a preferred embodiment the capsule is sealed by "banding". In another preferred embodiment the capsule is a soft gel capsule of appropriate material. In another preferred embodiment the drug solution is adsorbed on a pharmaceutically acceptable carrier and the drug is dosed as a powder in a capsule and in yet another preferred embodiment the powder is further compounded into a tablet form. In an embodiment of this aspect the composition of fenofibrate is dosed at a level of about 5 mg fenofibrate to 50 mg fenofibrate per day, more preferably about 10 mg to about 40 mg fenofibrate per day and most preferably about 30 to about 35 mg fenofibrate per day.

Another aspect of this invention encompasses the method of treating a patient for elevated triglyceride levels comprising dosing a composition of fenofibrate that comprises a therapeutically effective amount of fenofibrate that is dissolved in menthol and further comprises at least one surface active agent. In one embodiment the composition is dosed as a viscous solution in a capsule. In one embodiment this capsule is a hard gelatin capsule or equivalent. In a preferred embodiment the capsule is sealed by "banding". In another preferred embodiment the capsule is a soft gel capsule of appropriate material. In another preferred embodiment the drug solution is adsorbed on a pharmaceutically acceptable carrier and the drug is dosed as a powder in a capsule and in yet another preferred embodiment the powder is further compounded into a tablet form. In an embodiment of this aspect the composition of fenofibrate is dosed at a level of about 5 mg fenofibrate to 50 mg fenofibrate per day, more preferably about 10 mg to about 40 mg fenofibrate per day and most preferably about 30 to about 35 mg fenofibrate per day.

Another aspect of this invention encompasses the method of treating a patient for elevated triglyceride levels comprising dosing a composition of fenofibrate that comprises a therapeutically effective amount of fenofibrate that is dissolved in PEG 1000 and Poloxamer 407. In one embodiment the composition is dosed as a viscous solution in a capsule. In one embodiment this capsule is a hard gelatin capsule or equivalent. In a preferred embodiment the capsule is sealed by "banding". In another preferred embodiment the capsule is a soft gel capsule of appropriate material. In another preferred embodiment the drug solution is adsorbed on a pharmaceutically acceptable carrier and the drug is dosed as a powder in a capsule and in yet another preferred embodiment the powder is further compounded into a tablet form. In an embodiment of this aspect the composition of fenofibrate is dosed at a level of about 10 mg fenofibrate to 100 mg fenofibrate per day, more preferably about 30 mg to about 70 mg fenofibrate per day, and most preferably about 65 mg fenofibrate per day.

### Example 1. Fenofibrate Formulations in Menthol

Formulations were prepared by heating menthol to about 60° C while stirring and adding the additives. The mixture was stirred until all the components dissolved to form a melt. Thereafter, the melt was cooled to about 50° C, fenofibrate was added and stirred until dissolved, the mixture was cooled to room temperature, and dispensed into capsules. The formulations made, on a per capsule basis are listed in Table 1 and Table 2.

**Table 1 . Formulations of Fenofibrate in Menthol and Tween**

| **Formulation** | **117.52.2** | **117.52.3** | **117.55.2** | **160.16** |
|---|---|---|---|---|
| Material | mg (%) | mg (%) | mg (%) | mg (%) |
| Fenofibrate | 54 (25.2) | 54 (20.5) | 54 (12.4) | 10 (7.7) |
| Menthol | 50 (23.4) | 100 (37.9) | 80 (18.4) | 25 (19.2) |
| Na Ducosate | 25 (11.7) | 25 (9.5) | 0 | 10 (7.7) |
| Tween 80 | 85 (39.7) | 85 (32.2) | 300 (69.1) | 85 (65.4) |
| **Total** | **214 (100)** | **264 (100)** | **434 (100)** | **130 (100)** |

**Table 2. Formulations of Fenofibrate in Menthol and Cremophor**

| **Formulation** | **117.55.3** | **117.55.6** |
|---|---|---|
| Material | mg (%) | mg (%) |
| Fenofibrate | 54 (12.4) | 54 (10.9) |
| Menthol | 80 (18.4) | 80 (16.2) |
| Na Ducosate | 0 | 40 (8.1) |
| Cremophor | 300 (69.1) | 300 (60.7) |
| Glycerine | 0 | 20 (4.0) |
| **Total** | **434 (100)** | **494 (100)** |

The formulations were tested for their small volume in vitro release characteristics in 50 ml 0.1N HCl at 37° C and 150 rpm using the following procedure:

### III. Instrumentation (or equivalent)

a. Automated Dissolution System comprising:
   Hanson SR8 Plus Test Station
   Hanson Auto Plus Maximiser System Controller
   Hanson Auto Plus MultiFill Fraction Collector
b. HPLC System comprising:
   pump - Merck Hitachi L-7100
   autoinjector - Merck Hitachi L-7200
   column oven - Merck Hitachi L-7300
   detector - Merck Hitachi L-7400
   interface and integration software - Merck Hitachi D-7000

### RELEASE TEST PROCEDURE

| | |
|---|---|
| Equipment: | 6-vessel assembly, small volume vessels and paddles |
| Medium: | 0.1N HCl for 30 min. |
| Volume: | 50 ml |
| Stirring Rate: | 150 RPM |
| Temperature: | 37° C ± 0.5° C |

### 0.1N HCl Preparation

Dilute 8.5ml HCl 37% to 1 liter with purified water.

### Procedure

Place one weighed capsule in each vessel containing 0.1N HCl and immediately operate the apparatus for 30 min.
Unless otherwise specified, 3 ml samples are withdrawn from each vessel and immediately filtered through PTFE membranes at 15 and 30 min.

### IV. Analysis Parameters

| | |
|---|---|
| Column & Packing: | Hypersil ODS BDS, 150X4.6mm, 5m |
| Column Temperature: | RT |
| Injector Temperature: | RT |
| Mobile Phase: | 40:60 dilute phosphoric acid: acetonitrile |
| Flow Rate: | 2.0 ml/min. |
| Detector: | UV at 286 nm |
| Sample / Injection Volume: | 10 µL |
| Injector Wash Solution: | 50:50 purified water: acetonitrile |

The small volume was chosen as a model of the conditions in a fasted stomach. The results of these tests are given in Table 3.

**Table 3. In vitro release of Fenofibrate in 50 ml 0.1N HCl**

| **Formulation** | **% dissolved 15 min** | **% dissolved 30 min** |
|---|---|---|
| 117.52.2 | 11.9 | 14.4 |
| 117.52.3 | 31.7 | 32.3 |
| 117.55.2 | 12.5 | 15.7 |
| 160.16 | 93.3 | 83.0 |
| 117.55.3 | 17.9 | 17.5 |
| 117.55.6 | 15.6 | 27.1 |
| Micronized Fenofibrate | 0 | 0 |

Under these conditions micronized fenofibrate gave no dissolution at all. Formulation 160.16 gave the best results under this model condition. The range of values for the 6 tablets were 90.3% to 99.3% at 15 minutes and 74.9% to 94.6% at 30 minutes. The occasional result of lower dissolution at longer time points may be caused by menthol leaching out of the presumed spontaneously formed micelle like structures which are believed to aid in dissolution and the subsequent precipitation of some of the material.

### Example 2. Fenofibrate Formulations in a Surfactant Mixture

Polyethylene glycol (PEG 1000) and poloxamer 407 were heated to 60° C while being stirred. Fenofibrate was added and the stirring continued until all had dissolved. The melt was dispensed into capsules and allowed to cool.

**Table 4. Formulations of Fenofibrate with PEG and Poloxamer**

| **Formulation** | **128.52.2** | **117.58.3** | **Micronized Fenofibrate** |
|---|---|---|---|
| Material | mg (%) | mg (%) | mg (%) |
| Fenofibrate | 54 (12.4) | 54 (35.1) | 54 (100) |
| Menthol | 0 | 0 | 0 |
| Na Ducosate | 0 | 0 | 0 |
| PEG 1000 | 80 (18.4) | 50 (32.5) | 0 |
| Poloxamer 407 | 300 (69.1) | 50 (32.5) | 0 |
| Cremophor | 0 | 0 | 0 |
| Glycerine | 0 | 0 | 0 |
| **Total** | **434 (100)** | **154 (100)** | **54 (100)** |

These two formulations were tested, along with untreated micronized fenofibrate, for their in vitro release in a USP type II dissolution tester using 900 ml water containing 0.5% SLS at 37° C and 50 rpm. Fenofibrate content of the solutions was determined by HPLC as described above. The results are shown in Table 5

**Table 5. Cumulative Release of Fenofibrate in 900 ml 0.5% SLS**

| **Formulation** | Micronized Fenofibrate | 128.52.2 | 117.58.3 |
|---|---|---|---|
| Time (min) | % released | % released | % released |
| 15 | 10.7 | 79.4 | 41.8 |
| 30 | 20.2 | 84.6 | 84.9 |
| 60 | 31.6 | 85.2 | 91.8 |

### Example 3. In Vivo Pharmacokinetics in Dogs

Two formulations were prepared as shown in Table 6. MAZ118 has the same formulation as 160.16 in Example 1. Formulation 107.69 is based on PEG 1000, poloxamer 407 as the formulations in Example 2 with the addition of a small amount (1%) of sodium ducosate and sorbitol as a solid carrier. In both cases the fenofibrate is in solution in the formulation.

**Table 6. Formulations used in the trial**

| **Formulation** | **MAZ118** | **107.69** |
|---|---|---|
| Material | mg (%) | mg (%) |
| Fenofibrate | 10 (7.7) | 9.9 (9.9) |
| Menthol | 25 (19.2) | 0 |
| Na Ducosate | 10 (7.7) | 1.0 (1.0) |
| Tween 80 | 85 (65.4) | 0 |
| Gelucire 33/01 | 0 | 6.6 (6.6) |
| PEG 1000 | 0 | 6.6 (6.6) |
| Poloxamer 407 | 0 | 9.9 (9.9) |
| sorbitol | 0 | 66.0 (66.0) |
| **Total** | **130 (100)** | **100 (100)** |

### Production of MAZ118

A double walled glass reactor was heated to 65°C. Menthol (EP), 50 grams, Tween 80 (Uniqema), 170 grams, and Ducosate Sodium (USP), 20 grams were added to the reactor. The mixture was stirred at 200 rpm until a melt solution was formed. Fenofibrate (Chemagis Ltd.), 20 grams, was added to the above melt and stirred at 200 rpm until full dissolution took place. The solution was cooled to 30° C. Capsules size "0" were filled with the melt solution, 130 mg ± 7 mg. The solution cooled to a viscous liquid. The capsules were found to have 10.5 mg ± 3.9% RSD of fenofibrate in a viscous liquid.

### Production of 107.69

A glass reactor was heated to 65°C. Gelucire 33/01 (Gattefosse), 1.0 grams, PEG 1000 (NF), 1.0 gram, Poloxamer 407 (BASF), 1.5 gram, and Ducosate Sodium (USP), 1.0 gram, were added to the reactor. The mixture was stirred at ~200 rpm until a melt solution was formed. Fenofibrate (Chemagis Ltd.), 1.5 grams, was added to the above melt and stirred at ~200 rpm until full dissolution took place. To the melt was added Sorbitol (NF), 10.0 grams , the mixture was mixed well and cooled. Tablets of 7 mm diameter and weighing 100 mg each were hand pressed in a Manesty F3 single punch tablet press. Each tablet had 9.9 mg of fenofibrate.

In vitro dissolution of these two formulations along with formulation 160.16 were carried out as follows:

| | |
|---|---|
| Equipment: | 6-vessel assembly, Apparatus 2/II (Paddle). |
| Medium: | 0.5% SLS for 1 hour |
| Volume: | 500 ml |
| Stirring Rate: | 50 RPM |
| Temperature: | 37°C±0.5°C |

The fenofibrate content of the samples was determined by HPLC as above.
The results of the dissolution test are shown in Table 7.

**Table 7. % Cumulative Dissolution of Fenofibrate**

| **Formulation** | 160.16 | | MAZ118 | | 107.69 | |
|---|---|---|---|---|---|---|
| Time (min) | % released | % released range (n=6) | % released | % released range (n=12) | % released | % released range (n=6) |
| 5 | 78.7 | 58.0 - 96.4 | n.m. | n.m. | 18.4 | 14.3 - 20.0 |
| 10 | 92.5 | 88.8-106.0 | n.m. | n.m. | 47.2 | 44.3 - 50.4 |
| 15 | n.m. | n.m. | 104.5 | 83.6 - 126.6 | n.m. | n.m |
| 20 | 93.5 | 83.3-102.4 | n.m | n.m | 70.9 | 67.5 - 76.5 |
| 30 | 96.7 | 89.9-106.9 | 104.5 | 98.0 - 112.7 | 78.0 | 73.0 - 80.4 |
| 60 | 94.2 | 87.5-103.7 | 105.0 | 99.0 - 112.4 | 82.5 | 79.8 - 86.4 |

| | | | | | | |
|---|---|---|---|---|---|---|
| n.m. = not measured | | | | | | |

### PK Trial in Dogs

The trial was conducted as an open-label, randomized, single-dose, 3-way crossover comparative bioavailability study. The study was designed to determine the AUC₀₋ₜ, AUC_{inf}, Cₘₐₓ, Tₘₐₓ and t_{½} for each formulation. The sample group consisted of six beagle dogs (five female and one male weighing about 10 kg each). Each dog was administered one of three treatments. The first treatment, treatment A, comprised of administering a hard gelatin capsule containing 10 mg fenofibrate formulation MAZ118; the second treatment, treatment B, comprised administration of a 1 x 54 mg fenofibrate tablet Tricor® (Abbott Laboratories); and the third treatment, treatment C, comprised of administration of a hard gelatin capsule containing 10 mg fenofibrate formulation 107.69. Each dog was administered a single oral dose with 10 ml water. After a two week washout the dogs were crossed over to another of the treatments.

Blood samples (4 ml) were collected in EDTA containing tubes before dosing and at 0.5, 1, 1.5, 2, 3, 4, 6, 8, and 24 hours post dosing. The samples were analyzed for fenofibric acid in plasma using an LC/MS/MS method validated for the range of 5 to 100 ng/ml.

### Results

The individual and average pharmacokinetic parameters for each of the two test sessions (A and C) compared to the reference session (B) are given in Tables 8 and 9.

**Table 8. Individual and Average Pharmacokinetic Parameters for Fenofibric Acid in Plasma for the Comparison of MAZ118 (Test) at a Dose of 10 Mg to Tricor® (Ref) at a Dose of 54 Mg**

| (Results of Fenofibrate PK Trial in Dogs) | | | | | | | |
|---|---|---|---|---|---|---|---|
| vol-sess | AUC₀₋ₜ (h*ng/g) | AUC_{inf} (h*ng/g) | t_{1/2}(h) | Tₘₐₓ (h) | Cₘₐₓ (ng/g) | Cₘₐₓₜₑₛₜ / C_{maxref} | AUC_{inftest} / AUC_{infref} |
| dog #1 (test) | 4670.7 | 4878.2 | 6.0 | 0.5 | 1068.2 | 1.26 | 0.90 |
| dog #2 (test) | 3413.9 | 4808.0 | 14.1 | 0.5 | 978.2 | 1.97 | 1.11 |
| dog #4 (test) | 4566.1 | 6232.3 | 15.0 | 1.0 | 1229.4 | 0.50 | 0.81 |
| dog #5 (test) | 5187.8 | 7245.2 | 16.8 | 1.0 | 2122.0 | 1.26 | 0.73 |
| dog #9 (Test) | 4041.0 | 5035.5 | 11.0 | 1.5 | 795.5 | 1.43 | 0.94 |
| dog #12 (test) | 7661.6 | 9588.4 | 11.3 | 1.0 | 1872.8 | 0.98 | |
| | | | | | | | |
| dog #1 (ref) | 4839.8 | 5394.7 | 7.3 | 0.5 | 847.7 | | |
| dog #2 (ref) | 3143.8 | 4321.6 | 13.4 | 0.5 | 497.4 | | |
| dog #4 (ref) | 6196.4 | 7698.1 | 12.0 | 0.5 | 2479.2 | | |
| dog#5 (ref) | 6745.4 | 9950.7 | 17.6 | 1.5 | 1688.3 | | |
| dog#9 (ref) | 3563.6 | 5367.5 | 16.8 | 0.5 | 554.9 | | |
| dog#12 (ref) | 9811.3 | | | 1.0 | 1917.8 | | |
| | | | | | | | |
| **AVG(test)** | **4923.5** | **6297.9** | **12.3** | **0.9** | **1344.3** | **1.232** | **0.899** |
| **AVG (ref)** | **5716.7** | **6546.5** | **13.4** | **0.8** | **1330.9** | | |
| | | | | | | | |
| **%CV (test)** | **29.87%** | **29.75%** | **31.04%** | **41.06%** | **39.48%** | | |
| **%CV (ref)** | **42.90%** | **34.65%** | **30.66%** | **55.78%** | **61.24%** | | |

**Table 9. Individual and Average Pharmacokinetic Parameters for Fenofibric Acid in Plasma for the Comparison of Formulation 107.69 (Test) at a Dose of 10 Mg to Tricor® (Ref) at a Dose of 54 Mg**

| (Results of Fenofibrate PK Trial in Dogs) | | | | | | | |
|---|---|---|---|---|---|---|---|
| vol-sess | AUCp₀₋ₜ (h*ng/g) | AUC_{inf} (h*ng/g) | t_{1/2} (h) | Tₘₐₓ (h) | Cₘₐₓ (ng/g) | Cₘₐₓₜₑₛₜ/ C_{maxref} | AUC_{inftest}/ AUC_{infref} |
| dog #1 (test) | 2224.0 | | | 0.5 | 441.8 | 0.52 | |
| dog #2 (test) | 2250.2 | 2598.7 | 9.4 | 0.5 | 508.4 | 1.02 | 0.60 |
| dog #4 (test) | 2918.4 | 3392.2 | 10.3 | 0.5 | 1019.3 | 0.41 | 0.44 |
| dog #5 (test) | 3242.3 | 3816.7 | 10.2 | 0.5 | 732.2 | 0.43 | 0.38 |
| dog #9 (test) | 2070.6 | 2436.3 | 9.6 | 0.5 | 471.9 | 0.85 | 0.45 |
| dog #12(test) | 2915.7 | 4163.4 | 15.0 | 1.0 | 523.9 | 0.27 | |
| | | | | | | | |
| dog #1 (ref) | 4839.8 | 5394.7 | 7.3 | 0.5 | 847.7 | | |
| dog #2 (ref) | 3143.8 | 4321.6 | 13.4 | 0.5 | 497.4 | | |
| dog #4 (ref) | 6196.4 | 7698.1 | 12.0 | 0.5 | 2479.2 | | |
| dog #5 (ref) | 6745.4 | 9950.7 | 17.6 | 1.5 | 1688.3 | | |
| dog #9 (ref) | 3563.6 | 5367.5 | 16.8 | 0.5 | 554.9 | | |
| dog #12 (ref) | 9811.3 | | | 1.0 | 1917.8 | | |
| | | | | | | | |
| **AVG (test)** | **2603.5** | **3281.5** | **10.9** | **0.6** | **616.2** | **0.585** | **0.470** |
| **AVG (ref)** | **5716.7** | **6546.5** | **13.4** | **0.8** | **1330.9** | | |
| | | | | | | | |
| **%CV (test)** | **18.48%** | **22.89%** | **21.56%** | **34.99%** | **36.09%** | | |
| **%CV (ref)** | **42.90%** | **34.65%** | **30.66%** | **55.78%** | **61.24%** | | |

Table 8 shows that the average AUC₀₋ₜ for MAZ118 was 4923 (ng*hr/ml) or 492 (ng*hr/ml) per mg while the average AUC₀₋ₜ for Tricor® was 5716 (ng*hr/ml) or 106 (ng*hr/ml) per mg. The bioavailability of the 10 mg MAZ118 test formulation as expressed by AUC₀₋ₜ was 86% that of the 54 mg Tricor®. On a per mg basis the MAZ118 test formulation was 4.6 times more bioavailable than the reference formulation. The corresponding values for AUC_{inf} for the samples where a terminal half life (t_{½}) could be calculated showed the MAZ118 test formulation to be 5.2 times as effective as the Tricor® on a per milligram basis (630 (ng*hr/ml) per mg compared to 121 (ng*hr/ml) per mg). The average of the ratios of the individual AUC_{inf} shows the 10 mg test formulation of MAZ118 to have 90% of the bioavailability of the 54 mg Tricor®. The average Cₘₐₓ for MAZ118 was 1344.3 (ng/ml) or 134 (ng /ml) per mg while the average Cₘₐₓ for Tricor® was 1330.9 (ng/ml) or 24.6 (ng/ml) per mg. The values for average Tₘₐₓ were similar, 0.9 hr for the test formulation and 0.8 hr for the reference. The terminal elimination half life was similar for each formulation being 12.3 hours for the test formulation and 13.4 hours for the Tricor® reference formulation. The variability of the test formulation MAZ118, as expressed by %CV, was lower than in the reference formulation for both the AUC parameters and the Cₘₐₓ parameter.

Table 9 shows that the average AUC₀₋ₜ for formulation 107.69 was 2603 (ng*hr/ml) or 260 (ng*hr/ml) per mg while the average AUC₀₋ₜ for Tricor® was 5716 (ng*hr/ml) or 106 (ng*hr/ml) per mg. The bioavailability of the 10 mg test formulation as expressed by AUC was 46% that of the 54 mg Tricor®. On a per mg basis the 107.69 test formulation was 2.4 times more bioavailable than the reference formulation. The corresponding values for AUC_{inf} for the samples where a terminal half life (t_{½}) could be calculated showed the 107.60 test formulation to be 2.7 times as effective as the Tricor® reference formulation on a per milligram basis (328 (ng*hr/ml) per mg compared to 121 (ng*hr/ml) per mg). The average of the ratios of the individual AUC_{inf} shows the 10 mg test formulation 107.69 to have 47% of the bioavailability of the 54 mg Tricor®. The average Cₘₐₓ for formulation 107.69 was 616.2 (ng/ml) or 62 (ng /ml) per mg while the average Cₘₐₓ for Tricor® was 1330.9 (ng/ml) or 24.6 (ng/ml) per mg. The values for average Tₘₐₓ were similar, 0.6 hr for the test formulation and 0.8 hr for the reference. The terminal elimination half life was similar for each formulation being 10.9 hours for the test formulation and 13.4 hours for Tricor® reference formulation. The variability of the test formulation 107.69, as expressed by %CV, was lower than in the reference formulation for all the PK parameters measured.

### Conclusion

Both test formulations were shown to be more bioavailable than the reference formulation on a per milligram basis. Formulation 107.69, a solublized form of fenofibrate, was about 2.5 times more bioavailable as the reference formulation. Formulation MAZ118, a solublized form of fenofibrate comprising menthol and a surfactant, was about 5 times as bioavailable on a per milligram basis.

### Example 4. Further Fenofibrate Formulations in a Surfactant Mixture

### Production Method

### A. Fenofibrate granulate

Menthol (1.333 kg) was melted in a glass reactor at 50° C with stirring. Fenofibrate (133.3 gm), Poloxamer 407 (Lutrol F127, 76 gm), and PEG 6000 (76 gm) were added. The menthol melt was stirred at 50° C until all the components had dissolved. Microcrystalline cellulose (Avicel PH 101, 106.7 gm) was added to the melt which was stirred until a uniform suspension was obtained.

The menthol melt was divided into three equal portions and poured into three trays (stainless steel, 0.133 m² each) that were cooled to -40° C for quick solidification of the menthol suspension. The solid material on the trays was removed and milled through a 2.5 mm screen using an Erweka mill. The obtained powder was again divided into three portions and returned to the trays. Menthol was removed from the material on the trays by sublimation in a high vacuum tray drier at 0.2 mbar 36° C for about 53 hours. The powder was removed from the trays and milled through a 1.6 mm screen using an Erweka mill. The powder so obtained was weighed (346.4 gm) for a yield of 88%.

### B. Fenofibrate Tablets (145 mg)

The fenofibrate granulate from step A was milled through a 0.8 mm screen using an Erweka mill. The milled granulate (336 gm) was added to a polyethylene bag (50 x 70 cm). Crospovidone (108 gm), sodium bicarbonate (72 gm) and citric acid anhydrous (72 gm) were added and the blend mixed for 5 minutes. Magnesium stearate (12 gm) was added to the bag and the blend mixed for a further 1/2 minute. The total amount of blend so obtained was 600 grams.

The blend was pressed into tablets on a Manesty F3 single punch tabletting machine using oval shaped (8.8 mm x 17.6 mm) normal concave punches. Tablet weight design was 785 mg ± 39.3 mg at a hardness of 5 - 7 Kp. The tablets obtained had an average weight of 792 mg and a hardness of 6 Kp. Several batches were made and labeled MAZ149B, MAZ149B1 and MAZ149B2.

### In Vitro Release

The release of fenofibrate from the tablets was tested using a USP type II dissolution tester filled with 1000 ml 0.5% sodium lauryl sulfate (SLS) (w/v) in water at 37° C and 50 revolutions per minute (rpm). The amount of fenofibrate in each sample was determined by HPLC as above. The results are given in tables 10-12 for three batches.

**Table 10. Results of in vitro release of fenofibrate (% label claim). Batch MAZ149B**

| Time(min) | Vessel 1 | Vessel 2 | Vessel 3 | Vessel 4 | Vessel 5 | Vessel 6 | Vessel 7 |
|---|---|---|---|---|---|---|---|
| 10 | 80.2 | 69.2 | 75.2 | 76.2 | 78.0 | 75.5 | 71.8 |
| 15 | 92.7 | 86.9 | 90.5 | 90.5 | 90.8 | 88.6 | 87.5 |
| 30 | 99.3 | 94.8 | 96.6 | 97.7 | 96.9 | 93.0 | 95.6 |
| | | | | | | | |

| Time(min) | Vessel 8 | Vessel 9 | Vessel 10 | Vessel 11 | Vessel 12 | Avg | %RSD |
|---|---|---|---|---|---|---|---|
| 10 | 80.1 | 75.2 | 73.9 | 80.5 | 80.3 | 76.3 | 4.75 |
| 15 | 89.6 | 88.9 | 88.3 | 89.1 | 89.2 | 89.4 | 1.76 |
| 30 | 95.2 | 95.4 | 94.6 | 95.4 | 94.4 | 95.7 | 1.74 |

**Table 11. Results of in vitro release of fenofibrate (% label claim), Batch MAZ149B1**

| Time(min) | Vessel 1 | Vessel 2 | Vessel 3 | Vessel 4 | Vessel 5 | Vessel 6 | Avg | %RSD |
|---|---|---|---|---|---|---|---|---|
| 10 | 58.6 | 55.8 | 51.4 | 55.2 | 51.5 | 61.5 | 55.7 | 7.11 |
| 15 | 79.5 | 77.3 | 73.6 | 77.5 | 74.4 | 79.2 | 76.9 | 3.16 |
| 30 | 90.7 | 88.9 | 86.5 | 88.5 | 89.1 | 89.5 | 88.9 | 1.56 |

**Table 12. Results of in vitro release of fenofibrate (% label claim), Batch MAZ149B2**

| Time(min) | Vessel 1 | Vessel 2 | Vessel 3 | Vessel 4 | Vessel 5 | Vessel | Avg 6 | %RSD |
|---|---|---|---|---|---|---|---|---|
| 10 | 69.0 | 66.3 | 68.9 | 71.0 | 72.4 | 63.8 | 68.6 | 4.54 |
| 15 | 80.2 | 79.6 | 81.1 | 81.1 | 81.0 | 77.6 | 80.1 | 1.72 |
| 30 | 86.9 | 87.8 | 88.0 | 87.1 | 87.1 | 86.9 | 87.3 | 0.56 |

### Pharmacokinetic Trial in Humans

### Pharmacokinetic Test of MAZ149B and Tricor® 145 mg

A four way crossover bioequivalence pharmacokinetic trial was carried out in 12 healthy volunteers using MAZ149B (145 mg) and Tricor® (145 mg) as two of the arms. The other two arms were other test formulations. A one week washout was taken between each arm. Blood samples were taken at 0, 1, 2, 3, 3.5, 4, 4.5, 5, 5.5, 6, 6.5, 7, 8, 9, 10, 12, 16, 24 and 48 hours (19 samples per trial) and analyzed for fenofibric acid by a validated method. The 4 arm trials were carried out in both the fasted and fed states.

### Results

In the fasted state data was obtained for volunteers 1- 11 for the test MAZ149B (N=11) and for volunteers 2-11 for the reference Tricor® (N-10). The results are shown in table 13. The average values showed the bioavailability of the test to be 97.4% of the reference based on AUC₀₋ₜ (175334 vs. 180010 h*ng/g) and 97.7% of the reference based on AUC_{inf} (213653 vs. 218628 h*ng/g). The corresponding geometric mean values showed 97.5% based on AUC₀₋ₜ (169481 vs. 173880 h*ng/g) and 97.5% based on AUC_{inf} (205217 vs. 210558 h*ng/g). The geometric mean of the ratio of the individual ratios of test to reference AUC_{inf} was 1.006. The average values for Cₘₐₓ showed the test to be 99% of the reference (10570 vs. 0624 ng/g) and the geometric mean to be 100.7% (10340 vs. 10270 ng/g). The geometric mean of the ratios of the test to reference of the individual volunteers was 1.021. The variability of the bioavailability was very similar, 28.95% vs. 27.16% for %CV of the AUC₀₋ₜ values. %CV is coefficient of variance, which is the standard deviation expressed as percent of the arithmetic mean. The average terminal half life was 20.0 hours for the test product and 19.9 hours for the reference, while the average Tₘₐₓ was 2.5 hours for the test and 2.1 hours for the reference. The two formulations are bioequivalent in the fasted state. The two formulations are bioequivalent in the fasted state in that they fall within the range of 80-125% of each other in terms of AUC_{inf} and Cₘₐₓ. In fact, the two formulations are very close to 100% of each other in both of these pharmacokinetic parameters.

In the fed state, data was obtained for volunteers 1-5, 7-10 and 12 (N=10) for both the test MAZ149B and the Tricor® reference product. The results are collected in table 14. The average values showed that the bioavailability of the test to be 107.1 % of the reference based on AUC₀₋ₜ (150511 vs. 140627 h*ng/g) and 112.0% of the reference based on AUC_{inf} (185149 vs. 165310 h*ng/g). The corresponding geometric mean values showed 106.8% based on AUC₀₋ₜ (145402 vs. 136134 h*ng/g) and 111.2% based on AUC_{inf} (174021 vs. 156459 h*ng/g). The geometric mean of the ratio of the individual ratios of test to reference AUC_{inf} was 1.112. The average values for Cₘₐₓ showed the test to be 79.0 % of the reference (7557 vs.9567 ng/g) and the geometric mean to be 77.5% (7147 vs. 9217 ng/g). The geometric mean of the ratios of the test to reference of the individual volunteers was 0.775. The variability of the bioavailability was very similar, 27.16% vs. 26.41% for %CV of the AUC₀₋ₜ values. The average terminal half life was 17.4 hours for the test product and 16.1 hours for the reference, while the average Tₘₐₓ was 8.0 hours for the test and 3.6 hours for the reference. The improved bioavailability coupled with the lower Cₘₐₓ and later Tₘₐₓ indicate an improved product in the fed state (more efficacy; less side effects; and longer duration of action).

**Table 13. PK of MAZ149B* vs. Tricor®* in Fasted State (10536004)**

| Volunteer | AUC₀₋₄₈ₕ (h·ng/g) | AUC_{inf} (h·ng/g) | T_{1/2} (h) | Tₘₐₓ (h) | Cₘₐₓ (ng/g) | Cₘₐₓₜₑₛₜ/ C_{maxref} | AUC_{inftest}/ AUC_{infref} |
|---|---|---|---|---|---|---|---|
| 1 (test) | 133053.0 | 149267.0 | 15.5 | 3.0 | 9010.0 | | |
| 2 (test) | 166526.0 | 189578.0 | 17.1 | 2.0 | 12699.0 | 1.04 | 0.79 |
| 3 (test) | 142995.0 | 193559.0 | 25.8 | 2.0 | 6357.0 | 0.91 | 0.94 |
| 4 (test) | 121367.0 | 134750.0 | 15.4 | 2.0 | 9360.0 | 0.95 | 1.11 |
| 5 (Test) | 156850.0 | 176988.0 | 16.8 | 2.0 | 10489.0 | 0.87 | 0.92 |
| 6 (test) | 142581.0 | 167086.0 | 18.1 | 1.5 | 8397.0 | 1.39 | 1.09 |
| 7 (test) | 142733.0 | 201134.0 | 28.6 | 4.5 | 10952.0 | 1.15 | 1.12 |
| 8 (test) | 209259.0 | 251532.0 | 19.7 | 1.5 | 11138.0 | 0.74 | 0.94 |
| 9 (test) | 191656.0 | 227307.0 | 18.3 | 3.0 | 11300.0 | 1.13 | 1.05 |
| 10 (test) | 287539.0 | 345390.0 | 19.2 | 5.0 | 14627.0 | 1.09 | 1.16 |
| 11 (test) | 234120.0 | 313588.0 | 25.1 | 1.5 | 11944.0 | 1.08 | 1.01 |
| 12 (test) | | | | | | | |
| | | | | | | | |
| 1 (ref) | | | | | | | |
| 2 (ref) | 199790.0 | 239900.0 | 19.2 | 3.0 | 12242.0 | | |
| 3 (ref) | 150941.0 | 206776.0 | 25.8 | 1.5 | 6966.0 | | |
| 4 (ref) | 113223.0 | 121099.0 | 13.1 | 2.0 | 9894.0 | | |
| 5 (ref) | 166239.0 | 193362.0 | 17.5 | 4.0 | 12085.0 | | |
| 6 (ref) | 117039.0 | 153347.0 | 23.7 | 2.0 | 6020.0 | | |
| 7 (ref) | 151310.0 | 179099.0 | 18.7 | 1.0 | 9537.0 | | |
| 8 (ref) | 218602.0 | 267224.0 | 21.4 | 1.5 | 15025.0 | | |
| 9 (ref) | 188187.0 | 217502.0 | 16.7 | 2.5 | 10026.0 | | |
| 10 (ref) | 257562.0 | 298831.0 | 18.4 | 1.5 | 13396.0 | | |
| 11 (ref) | 237204.0 | 309136.0 | 24.1 | 2.0 | 11052.0 | | |
| 12 (ref) | | | | | | | |
| | | | | | | | |
| **AVG(test)** | **175334.5** | **213652.6** | **20.0** | **2.5** | **10570.3** | **1.035** | **1.012** |
| **AVG(ref)** | **180009.7** | **218627.6** | **19.9** | **2.1** | **10624.3** | | |
| | | | | | | | |
| **Geomn(test)** | **169481.3** | **205216.6** | **19.5** | **2.3** | **10339.7** | **1.021** | **1.006** |
| **Geomn(ref)** | **173879.9** | **210558.3** | **19.5** | **2.0** | **10270.3** | | |
| | | | | | | | |
| **std dev(test)** | **50758** | **66323** | **4.48** | **1.21** | **2241.80** | **0.18** | **0.12** |
| **std dev(ref)** | **48896** | **61060** | **3.88** | **0.88** | **2761.61** | | |
| | | | | | | | |
| **%CV (test)** | **28.95%** | **31.04%** | **0.22** | **0.48** | **0.21** | | |
| **%CV (ref)** | **27.16%** | **27.93%** | **0.20** | **0.42** | **0.26** | | |

| | | | | | | | |
|---|---|---|---|---|---|---|---|
| *Dose = 145 mg fenofibrate for MAZ149B (test) and Tricor (ref) "AVG" is arithmetic mean. "geomn" is geometric mean. "std dev" is standard deviation. | | | | | | | |

**Table 14. PK of MAZ149B* vs. Tricor®* in Fed State (10536005)**

| Volunteer | AUC₀₋₄₈ₕ (h·ng/g) | AUC_{inf} (h·ng/g) | T_{1/2} (h) | Tₘₐₓ (h) | Cₘₐₓ (ng/g) | Cₘₐₓₜₑₛₜ/ C_{maxref} | AUC_{inftest}/ AUC_{infref} |
|---|---|---|---|---|---|---|---|
| 1 (test) | 151598.0 | 201715.0 | 23.6 | 5.5 | 6517.0 | 1.01 | 1.15 |
| 2 (test) | 119863.0 | 126328.0 | 11.8 | 2.0 | 10583.0 | 0.87 | 1.10 |
| 3 (test) | 91601.0 | 97182.0 | 10.5 | 9.0 | 5224.0 | 0.76 | 1.05 |
| 4 (test) | 159339.0 | 202949.0 | 20.2 | 5.0 | 6725.0 | 0.86 | 1.14 |
| 5 (Test) | 183511.0 | 217445.0 | 18.6 | 3.5 | 14538.0 | 1.06 | 1.10 |
| 6 (test) | | | | | | | |
| 7 (test) | 106779.0 | 115154.0 | 11.9 | 4.5 | 5250.0 | 0.62 | 1.06 |
| 8 (test) | 195150.0 | 251495.0 | 19.9 | 12.0 | 7083.0 | 0.65 | 0.95 |
| 9 (test) | 119423.0 | 129598.0 | 12.8 | 4.5 | 6285.0 | 0.60 | 1.11 |
| 10 (test) | 217512.0 | 308070.0 | 24.1 | 10.0 | 8236.0 | 0.66 | 1.32 |
| 11 (test) | | | | | | | |
| 12 (test) | 160332.0 | 201554.0 | 20.6 | 24.0 | 5126.0 | 0.81 | 1.18 |
| | | | | | | | |
| 1 (ref) | 144704.0 | 175441.0 | 17.4 | 5.5 | 6441.0 | | |
| 2 (ref) | 110211.0 | 115275.0 | 11.4 | 2.0 | 12129.0 | | |
| 3 (ref) | 87114.0 | 92310.0 | 12.0 | 2.0 | 6898.0 | | |
| 4 (ref) | 147743.0 | 178775.0 | 18.7 | 3.0 | 7775.0 | | |
| 5 (ref) | 170745.0 | 197209.0 | 16.8 | 4.5 | 13670.0 | | |
| 6 (ref) | | | | | | | |
| 7 (ref) | 103810.0 | 108394.0 | 10.7 | 3.0 | 8532.0 | | |
| 8 (ref) | 200926.0 | 264258.0 | 22.8 | 3.0 | 10912.0 | | |
| 9 (ref) | 111382.0 | 116530.0 | 11.2 | 2.0 | 10440.0 | | |
| 10 (ref) | 182589.0 | 233712.0 | 23.1 | 2.0 | 12522.0 | | |
| 11 (ref) | | | | | | | |
| 12 (ref) | 147041.0 | 171197.0 | 16.9 | 9.0 | 6350.0 | | |
| | | | | | | | |
| **AVG(test)** | **150510.8** | **185149.0** | **17.4** | **8.0** | **7556.7** | **0.790** | **1.116** |
| **AVG(ref)** | **140626.5** | **165310.1** | **16.1** | **3.6** | **9566.9** | | |
| | | | | | | | |
| **geomn(test)** | **145402.2** | **174020.6** | **16.7** | **6.3** | **7146.7** | **0.775** | **1.112** |
| **geomn(ref)** | **136133.6** | **156458.7** | **15.5** | **3.2** | **9217.4** | | |
| | | | | | | | |
| **std dev(test)** | **40872** | **67163** | **5.16** | **6.44** | **2956.04** | **0.16** | **0.09** |
| **std dev(ref)** | **37136** | **57016** | **4.66** | **2.23** | **2712.70** | | |
| | | | | | | | |
| **%CV (test)** | **27.16%** | **36.28%** | **0.30** | **0.80** | **0.39** | | |
| **%CV (ref)** | **26.41%** | **34.49%** | **0.29** | **0.62** | **0.28** | | |

| | | | | | | | |
|---|---|---|---|---|---|---|---|
| *Dose = 145 mg fenofibrate for MAZ 149B (test) and Tricor (ref) "AVG" is arithmetic mean. "geomn" is geometric mean. "std dev" is standard deviation. | | | | | | | |

## Claims

1. A pharmaceutical composition comprising a fibrate drug in intimate association with a surfactant mixture comprising PEG 6000 and Poloxamer 407.

2. The composition of claim 1, wherein the fibrate drug is fenofibrate.

3. The composition of claim 1 or 2, wherein when the composition is formulated into a tablet, the tablet having a dissolution rate, as measured using a rotating blade method at 50 rpm, in 1000 ml of a dissolution medium constituted by water with 0.5% sodium lauryl sulfate at 37°C, such that at least about 51% is released in 10 minutes.

4. The composition of claim 3, wherein the dissolution rate is such that about 51-81% is released in 10 minutes.

5. The composition of claim 1,2 or 3, wherein the dissolution rate is such that at least about 73% is released in 15 minutes.

6. The composition of any of claims 1-4, wherein the dissolution rate is such that about 73-93% is released in 15 minutes.

7. The composition of claim 5, wherein the dissolution rate is such that at least about 85% is released in 30 minutes.

8. The composition of claim 6, wherein the dissolution rate is such that about 85-99% is released in 30 minutes.

9. The composition of claim 4, wherein the dissolution rate is such that about 71-81% is released in 10 minutes, about 86-93% is released in 15 minutes and about 93-100% is released in 30 minutes.

10. The composition of claim 2, wherein when the composition is formulated into a tablet containing 145 mg fenofibrate and when the tablet is orally administered to a human subject in fed state, the AUC₀₋₄₈ₕ ranges from about 91600 h.ng/g to about 217500 h.ng/g.

11. The composition of claim 10, the average AUC₀₋₄₈ₕ is about 150500 h.ng/g.

12. The composition of claim 2, wherein when the composition is formulated into a tablet containing 145 mg fenofibrate and when the tablet is orally administered to a human subject in fed state, the AUC_{inf} ranges from 97200 h·ng/g to about 308100 h·ng/g.

13. The composition of claim 12, wherein the average AUC_{inf} is about 185200 h·ng/g.

14. The composition of claim 2, wherein when the composition is formulated into a tablet containing 145 mg fenofibrate and when the tablet is orally administered to a human subject in a fed state, the AUC_{inf} is about 80-125% of the AUC_{inf} achievable with a 145 mg Tricor tablet administered to the subject in the fed state.

15. The composition of claim 14, wherein the AUC_{inf} is about 100% of the AUC_{inf} achievable with the 145 mg Tricor formulation administered to the subject in a fed state.

16. The composition of claim 2, wherein when the composition is formulated into a tablet containing 145 mg fenofibrate and when the tablet is orally administered to a human subject in a fasted state, the AUC₀₋₄₈ₕ ranges from about 121400 h.ng/g to about 287500 h.ng/g.

17. The composition of claim 16, wherein the average AUC₀₋₄₈ₕ is about 175300 h.ng/g.

18. The composition of claim 2, wherein when the composition is formulated into a tablet containing 145 mg fenofibrate and when the tablet is orally administered to a human subject in a fasted state, the AUC_{inf} ranges from about 134800 h·ng/g to about 345400 h·ng/g.

19. The composition of claim 18, wherein the average AUC_{inf} is about 213700 h·ng/g.

20. The composition of claim 2, wherein when the composition is formulated into a tablet containing 145 mg fenofibrate and when the tablet is orally administered to a human subject in a fasted state, the AUC_{inf} is about 80- 125% of the AUC_{inf} achievable with a 145 mg Tricor tablet administered to the subject in the fasted state.

21. The composition of claim 20, wherein the AUC_{inf} is about 100% of the AUC_{inf} achievable with the 145 mg Tricor formulation administered to the subject in the fasted state.

22. The composition of claim 2, wherein when the composition is formulated into a tablet containing 145 mg fenofibrate and when the tablet is orally administered to a human subject in a fasted state, the Cₘₐₓ ranges from about 6300 ng/g to about 14700 ng/g.

23. The composition of claim 22, wherein the average Cₘₐₓ is about 10600 ng/g.

24. The composition of claim 2, wherein when the composition is formulated into a tablet containing 145 mg fenofibrate and when the tablet is orally administered to a human subject in a fasted state, the Cₘₐₓ is about 80 - 125% of the Cₘₐₓ achievable with a 145 mg Tricor tablet administered to the subject in the fasted state.

25. The composition of claim 24, wherein the Cₘₐₓ is about 100% of the Cₘₐₓ achievable with a 145 mg Tricor tablet administered to the subject in the fasted state.

26. The composition of any preceding claim, comprising
between about 15% and about 25% by weight of fenofibrate,
between about 7% and about 13% by weight of PEG 6000, and
between about 7% and about 13% by weight of Poloxamer 407.

27. The composition of claim 26, further comprising at least one pharmaceutical acceptable carrier.

28. The composition of claim 27, wherein the pharmaceutical acceptable carrier is selected from water soluble (hydrosoluble) carriers such as sucrose, lactose or sorbitol or water insoluble carriers such as starch, cellulose, microcrystalline cellulose, or calcium phosphate.

29. The composition of claim 26, 27 or 28, further comprising at least one pharmaceutical disintegrant.

30. The composition of claim 29, wherein the at least one disintegrant is selected from the group consisting of crospovidone, croscarmellose, a bicarbonate salt, an organic acid, and combinations thereof.

31. The composition of claim 30, wherein the organic acid is chosen from the group consisting of citric acid and tartaric acid.

32. The composition according to any of claims 26-31 comprising about 19% by weight of fenofibrate, about 10.9% by weight of Poloxamer 407, about 10.9% by weight of PEG 6000 by weight, about 15.3% by weight of microcrystalline cellulose, about 18% by weight of crospovidone, about 12% by weight of sodium bicarbonate and about 12% by weight of citric acid.

33. The composition according to any of claims 26-31, comprising about 19% by weight of fenofibrate, about 10.9% by weight of Poloxamer 407, about 10.9% by weight of PEG 6000 by weight, about 15.3% by weight of microcrystalline cellulose, about 18% by weight of crospovidone, about 12% by weight of sodium bicarbonate and about 12% by weight of tartaric acid.

34. A process for preparing a composition of any preceding claim 1, comprising
(a) providing melted menthol;
(b) mixing melted menthol with the fibrate drug and a surfactant mixture comprising PEG 6000 and Poloxamer 407 to dissolve at least part of the fibrate drug and the surfactant mixture in the menthol; and
(c) removing the menthol via sublimation to obtain a mixture as the pharmaceutical composition.

35. A composition comprising fenofibrate or another fibrate drug in intimate association with a surfactant mixture.

36. The composition of claim 35, wherein the surfactant mixture comprises polyethylene glycol and a poloxamer.

37. The composition of claim 36, wherein the composition has a dissolution of at least about 80% in about 30 minutes, as measured using a USP type II dissolution tester using 900 ml water containing 0.5% sodium lauryl sulfate at 37°C and 50 rpm.

38. The composition of any of claims 35 to 37, wherein the polyethylene glycol is PEG 1000 and the poloxamer is poloxamer 407.

39. The composition of any of claims 35 to 38 comprising (a) about 12.4% fenofibrate, about 18.4% PEG 1000, and about 69.1% poloxamer 407; (b) about 35.1% fenofibrate, about 32.5% PEG 1000, and about 32.5% poloxamer 407; or (c) about 9.9% fenofibrate, about 6.6% PEG 1000, about 9.9% poloxamer 407, about 1.0% sodium ducosate, about 6.6% Gelucire (33/01) and about 66.0% sorbitol, wherein the fenofibrate, PEG 1000, poloxamier 407, sodium ducosate and Gelucire are adsorbed on the sorbitol.

40. The composition of any of claims 35 to 39 wherein when the composition is administered to a dog weighing about 10 kg (a) at a dose of about 10 mg, an average AUC₀₋ₜ of fenofibric acid of about 2604 (ng*hr/ml) is achieved; (b) an average AUC₀₋ₜ of fenofibric acid of about 260 (ng*hr/ml) per mg is achieved; or (c) at a dose of about 10 mg, a Cmax of fenofibric acid of at least about 400 ng/ml and an average Cmax of about 600 ng/ml are achieved.

41. Use of a composition of any of claims 1 to 40 for the manufacture of a medicament for treating an elevated triglyceride level in a patient.

42. The use of claim 41, wherein a fenofibrate dose of 5 to 50 mg per day is administered.
